**Europäisches Patentamt**

**(19)** **European Patent Office**

**Office européen des brevets**

**(11)** Publication number : **0 282 545 B1**

**(12)** # EUROPEAN PATENT SPECIFICATION

**(45)** Date of publication of patent specification :
05.02.92 Bulletin 92/06

**(51)** Int. Cl.⁵ : **A61M 5/00**

**(21)** Application number : **87906118.2**

**(22)** Date of filing : **07.09.87**

**(86)** International application number :
**PCT/SE87/00398**

**(87)** International publication number :
**WO 88/01881 24.03.88 Gazette 88/07**

**(54)** CONNECTOR AND A DISPOSABLE ASSEMBLY UTILIZING SAID CONNECTOR.

**(30)** Priority : **18.09.86 SE 8603928**
**09.12.86 SE 8605259**

**(43)** Date of publication of application :
**21.09.88 Bulletin 88/38**

**(45)** Publication of the grant of the patent :
**05.02.92 Bulletin 92/06**

**(84)** Designated Contracting States :
**AT BE CH DE FR GB IT LI LU NL SE**

**(56)** References cited :
**WO-A-86/05683**

**(56)** References cited :
**DE-A- 1 766 152**
**US-A- 3 150 661**
**US-A- 3 826 260**
**US-A- 4 084 588**
**US-A- 4 516 967**

**(73)** Proprietor : **Kabi Pharmacia AB**
**S-751 82 Uppsala (SE)**

**(72)** Inventor : **MALMBORG, Bengt, Eber**
**Skanegatan 47**
**S-252 52 Helsingborg (SE)**

**(74)** Representative : **Thylén, Eva**
**c/o Kabi Pharmacia AB Box 941**
**S-251 09 Helsingborg (SE)**

## Description

The present invention relates generally to the handling of toxic substances and drugs, especially pharmacologically active, hazardous substances.

More particularly, the invention relates to a connector for a container having at one end an opening which is closed by means of a pierceable closure and which is surrounded by an outwardly directed flange, and having a plunger movable within the container and closely engaging the container inner wall, a toxic substance, e.g. a drug component, being enclosed between said plunger and said closure, for mixing with a liquid prior to administration.

The invention also relates to a disposable assembly comprising said container, said connector and, optionally, a container for the liquid, interconnected to form a unit.

Many different types of pharmaceuticals are hazardous and risky to handle. Medical staff exposed to these drugs when preparing mixtures for administration to patients must take many precautions to avoid the risks associated with the handling of the drugs. In view hereof, there is urgent need for devices which reduce the staff's exposure to such drugs and which could simplify their routines to a considerable extent.

Cytostatic drugs as used for the treatment of cancer are one type of drug which requires safe handling. These drugs are often supplied by the manufacturer in the form of a dry powder in a sealed glass vial together with an ampoule containing the solvent, for example water or an aqueous solution, for dissolving the drug. The most important steps which the medical staff must take in order to prepare a drug solution ready for administration, such as injection, to the patient, may be summarised as follows:

(1) opening an ampoule containg the solvent and drawing the solvent from the ampoule up into a syringe;

(2) carefully emptying the solvent to avoid excess pressure from the syringe into a vessel containing the dry cytostatic agent;

(3) drawing the dissolved drug, after a period of dissolution, carefully up into a syringe intended for the administration.

It is recommended that this preparation be carried out in a fume cupboard and a well-aerated room, and also that the work be done by specially trained personnel.

The preparation technique described also involves a risk of contamination, incorrect dosing and, furthermore, a risk that the wrong components are mixed, especially if mixing is effected by untrained staff members.

Many attempts at solving these problems have been made, for example by supplying the syringe and the ampoule connected together by means of a special connector. Examples of such combinations are disclosed in, inter alia, US-A-3,826,260, W0-A-86/05683 and DE-A-1,766,152.

The combination according to US-A-3,826,260 comprises a syringe containing a solvent, a vial containing a drug, and a connector including a cannula. The syringe is of the type having a neck and a seal, the seal being held in position by a specially designed sleeve of reduced diameter. The vial is of similar construction and has a seal and a specially designed sleeve. The cannula is mounted in a support which is movable from an outer to an inner position on the first-mentioned sleeve. Although this combination is intended for mixing a drug with a solvent, mixing takes place in the vial, not in the syringe. The specially designed sleeve of the syringe and the vial for connection with the cannula support and the connector, respectively, make this combination complicated and fairly long. Furthermore, the combination does not permit connection to an optional means of administration.

W0-A-86/05683 discloses a device for transferring two components of a drug between two containers and to the patient, respectively. The device comprises a connector having a single or double cannula. Two sleeve-shaped supports can be screwed onto the connector to protect the cannula. Different types of containers are insertable through the supports to a position in which the cannula ends pierce the seals at the container ends. In this manner, the component of one container can be transferred to the component of the other container. The device requires further protective means for the cannula ends in the event that the supports are not attached to the connector. However, also these protective means must be pierceable by the cannula points. When this device is to be used, the supports are interconnected with a connector, whereupon two containers, each with one component, can be interconnected via the cannula by inserting them into the supports in a direction toward one another. After the component of one container has been transferred to the other container, the empty container is removed and replaced by a container having a retractable plunger, and the two components which are now mixed, are transferred to the latter container prior to administration.

DE-A-1,766,152 discloses a device for converting a single-compartment syringe into a two-compartment syringe. The device comprises two sleeves, each having an open end and a closed end. Extending through the sealed end of one sleeve is a double-pointed cannula, and this sealed end also has an external thread for threaded engagement with an internal thread in the open end of the other sleeve. Each sleeve has a container with a seal immediately adjacent each cannula point. When the device is to be used, the sleeves are screwed together and the cannula with its two points pierces the seals of the containers. The components in the containers can now be mixed by retracting a plunger in one of the containers. Finally, the sleeves are unscrewed from one another to provide a syringe ready for administration of the mixed drug.

The object of the present invention is to provide a connector which is intended for containers with flanged ends and which satisfies the requirements stipulated for the handling of poisonous drugs, viz. that the staff handling the drug must not be exposed thereto during any phase of the handling, and that no contamination must occur. In addition, the connector should be simple, inexpensive and comprise but few parts, such that it can form part of a disposable assembly. Furthermore, the connector must be connectible to different means of administration.

This object is achieved, according to the present invention, in that the connector is in the form of a sleeve which is closed at one end, which is open at its opposite end to receive the flanged container end as a plunger, and which has internal positioning means cooperating with the flange and adapted to define an inserted and an extracted position of said sleeve relative to the flanged end of the container; a piercing member provided at the sealed end of the sleeve and directed towards the open sleeve end and adapted to pierce the closure of the container upon displacement of the sleeve from the retracted to the inserted position; and a connecting member provided at the closed sleeve end and directed opposite to said piercing member and connected therewith by means of a through passage, for connection of the substance or drug container first to a liquid container for sucking up liquid in said substance or drug container by retraction of the plunger therein, and then to a means for administration of the substance/liquid mixture.

The connector thus is a single element which cooperates directly with the outwardly directed flange on the container. Because of the positioning means according to the invention, the container can be safely moved between the two positions relative to the sleeve whereby, in particular, resealing of the container can be effected during the mixing of the drug component and the liquid by displacing the sleeve from the inserted to the extracted position in which the piercing member is outside the closure which thus can reseal the opening in the container end.

A similar construction is disclosed in the U S-A-3,150,661 which relates to a disposable combination hypodermic needle and cartridge or ampoule which is provided with means for maintaining the parts in sterile condition for transportation, storage and use, and in which the needle and ampoule are assembled at the factory for transportation and sale as a unit. The disposable cartridge-needle unit can be utilized with disposable syringes.

In contrast to this previously known combination the present invention concerns a connector, which is a single element and which comprises a partition from which the piercing member and the connecting member extend in opposite directions and through which members a through passage is provided. Thus, in contrast to the arrangement disclosed in the US-A-3,150,661 no needle and, consequently, no needle engagement means are required according to the present invention. From this follows that the connector according to the invention is cheaper and easier to manufacture and the connector can be used for different purposes, the most important of which are for handling poisonous drugs. It cannot be realized how the device according to US-A-3,150,661 can be used for these purposes.

Futhermore, the positioning means according to the present invention may be in the form of at least one inwardly directed bead on the inner side of the sleeve. In an especially advantageous embodiment, the connecting member also constitutes a piercing member for a pierceable closure of the liquid container. In this embodiment, the connector preferably has a sleeve extension extending in the same direction as the connecting member, for guiding the liquid container toward the connecting member. Internal positioning means may also be arrange in the sleeve extension to define an inserted and a retracted position of the extension relative to the liquid container.

Connection to the means of administration is simplified considerably if the extension is designed for breakaway removal from the sleeve.

The invention comprises not only the connector, but also a combination of the connector and the said drug container, as well as a combination of the connector, the said drug container and the said liquid container.

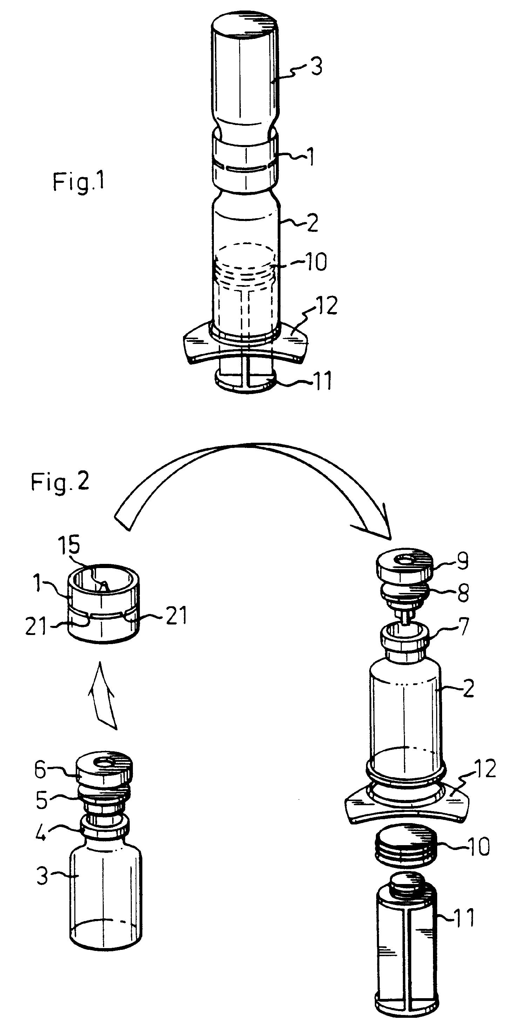

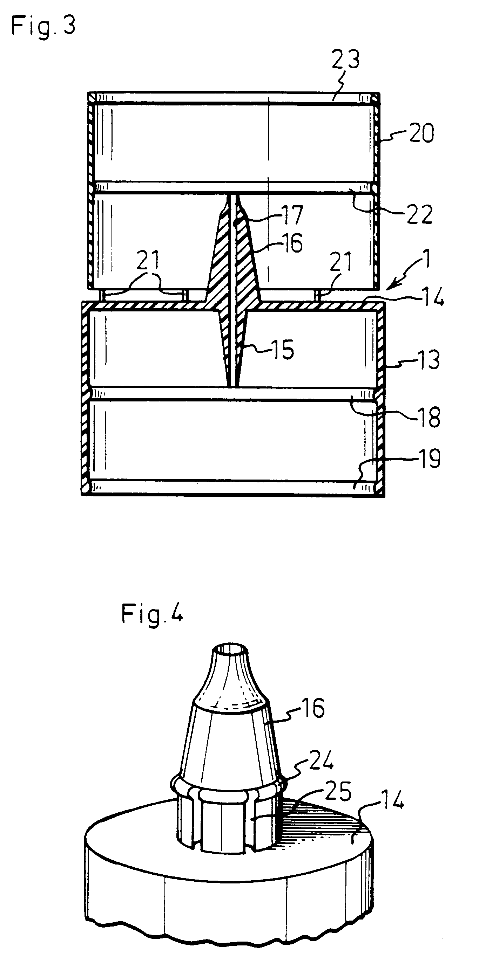

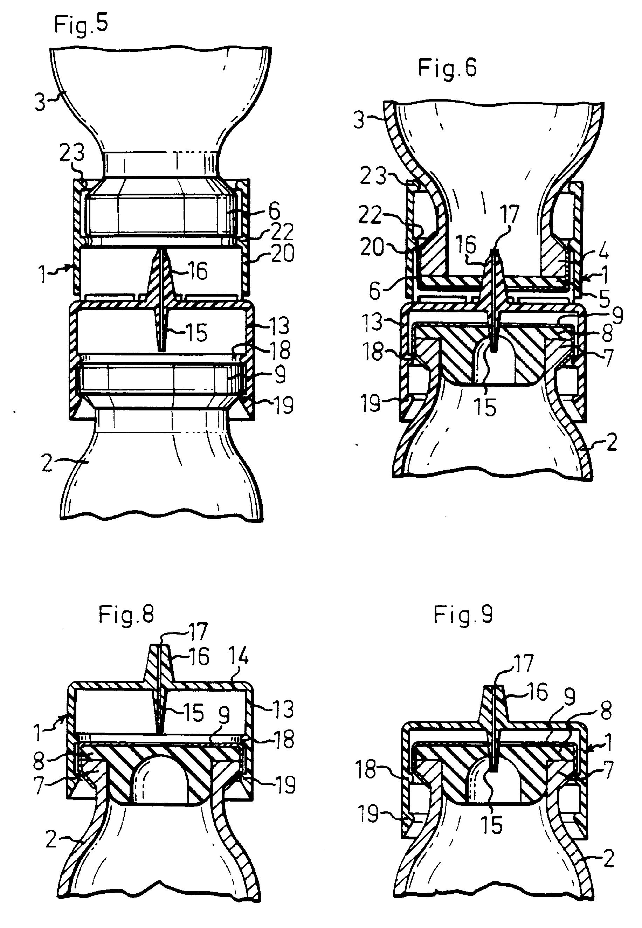

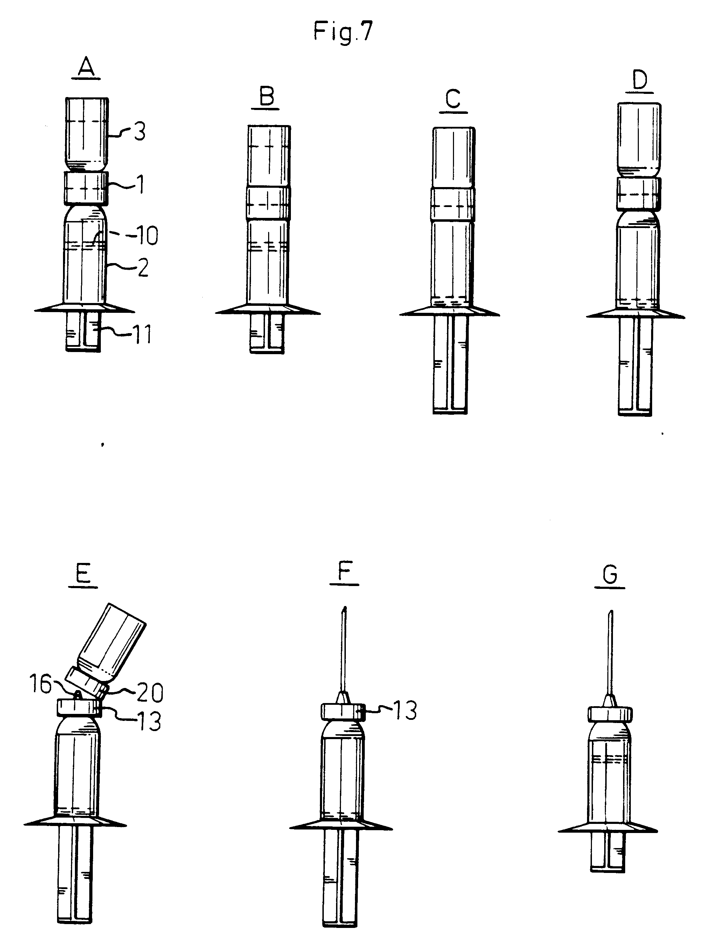

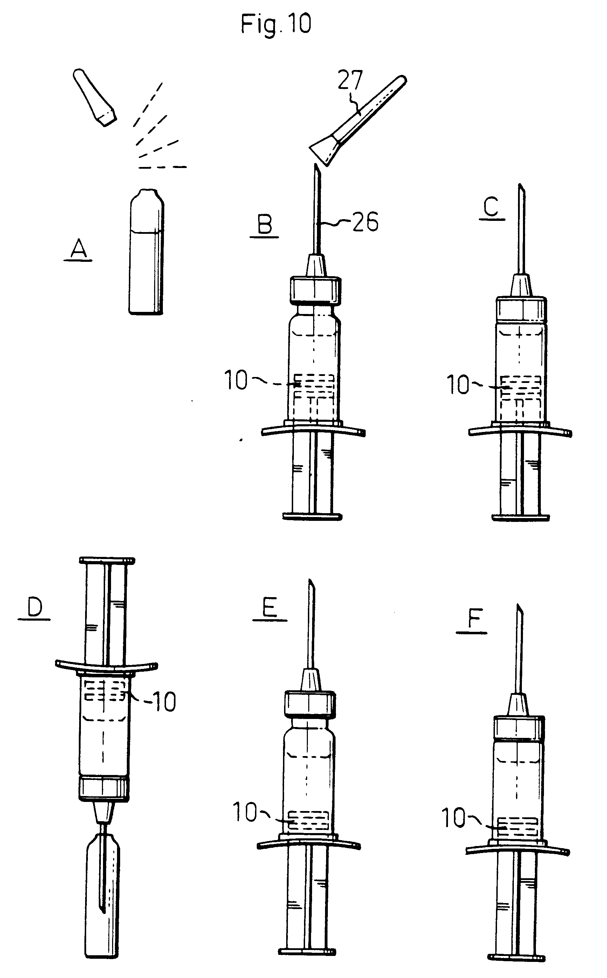

The invention will be described in more detail below, reference being had to the accompanying drawings. Fig. 1 illustrates a preferred embodiment of the disposable assembly according to the invention, comprising a drug container, a connector, and a liquid container. Fig. 2 is an exploded perspective view of the parts comprised by the disposable assembly according to Fig. 1. Fig. 3 is a longitudinal section of a connector according to the invention. Fig. 4 is a perspective view of the connecting and piercing members of the connector. Figs. 5 and 6 are longitudinal sections illustrating the retracted and inserted positions of the connector according to Fig. 3 relative to the containers according to Figs. 1 and 2. Fig. 7 illustrates the operating sequence during use of the disposable assembly according to Fig. 1. Figs. 8 and 9 illustrate an alternative connector in, respectively, a retracted and an inserted position relative to a drug container. Fig. 10 illustrates the operating sequence during use of the connector according to Figs. 8 and 9.

The disposable assembly shown in Figs. 1 and 2 comprises a connector 1, a container 2 for a drug component and a liquid container 3. The container 3 is a conventional vial having a neck and a flange 4 around the neck opening, a closure 5 in the form of, for example, a rubber stopper, and a cap 6 having a central hole and intended to keep the closure 5 in position in the neck opening by engaging the flange 4. Also the container 2 has a neck provided with a flange 7, a closure 8 and a cap 9. A plunger 10 is displaceable within the container 2 by means of a plunger rod 11, insertion of the plunger 10 being simplified by a finger grip 12 on the container 2. The plunger 10 and the plunger rod 11 are disconnectible from one another by means of a threaded connection.

In manufacturing the disposable assembly according to Fig. 1, a drug component is first introduced into the container 2, with the plunger 10 partly inserted therein, whereupon the container 2 is closed by means of the closure 8 which may be a stopper suitable for freeze-drying and is secured in position by means of the cap 9. The liquid to be admixed to the drug component of the container 2 is filled into the container 3 which then is closed by means of the closure 5 and the cap 6. After that, the containers 2 and 3 are interconnected by means of the connector 1, the container 2 and also the container 3 being fixed in a retracted position relative to the connector 1 by cooperation between the flange 4, 7 and internal positioning means in the connector 1.

The embodiment of the connector 1 as illustrated in Fig. 3, which preferably is made of plastic, comprises a sleeve 13 having an open end and another end closed by means of a partition 14. Extending from the end of the partition 14 toward the open end of the sleeve 13, is a piercing member 15 in the form of a truncated cone. A connecting member 16 extends from the partition 14 in the opposite direction to the piercing member 15. The connecting member 16 is conical along the major part of its length and thus can constitute one part of a so-called luer coupling. A through passage 17 communicates the piercing member 15 and the connecting member 16 and thus extends also through the partition 14. More particularly, the passage 17 opens into the free end of the piercing member 15 and the connecting member 16. A sharp cutting edge is formed around the opening of the passage 17 into the piercing member 15. A similar sharp cutting edge is formed around the opening of the passage 17 into the connecting member 16 which thus also constitutes a piercing member. The sleeve 13 has a first inner bead 18 approximately on a level with the point of the piercing member 15. A second bead 19 is formed at the open end of the sleeve 13.

The connector 1 shown in Fig. 3 furthermore has an extension 20 of the sleeve 13, said extension 20 extending in the same direction as the connecting member 16. Also the extension 20 is sleeve-shaped, although not connected with the sleeve 13 along its entire circumference, but only within limited portions 21. Like the sleeve 13, the extension 20 furthermore has an inner bead 22 approximately on a level with the point of the connecting member 16, as well as a bead 23 at its open outer end.

Fig. 4 illustrates another embodiment of the connecting member 16. Here, the portion of the connecting member 16 nearest the partition 14 is cylindrical and delimited from the conical portion by a collar 24. Axial grooves 25 extend along the cylindrical portion from the partition 14 and past the collar 24.

In Fig. 5, the connector 1 is in its retracted position relative to both the container 2 and the container 3. More particularly, the flange 7 of the container 2 is accommodated by the space defined by the inner side of the sleeve 13 and the beads 18 and 19. The container 2 is insertable into this position past the bead 19 because the material of the sleeve 13 is resilient. Similarly, the container 3 and its flange 4 are accommodated by the space which is formed in the extension 20 between the beads 22 and 23. In the positions indicated, the point of the piercing member 15 lies outside the rubber stopper 8 in the neck of the container 2, and the point of the connecting member 16 lies outside the closure 5 in the neck of the container 3.

In order to establish communication between the containers 2 and 3 via the passage 17, the containers 2 and 3 are displaced simultaneously or consecutively toward one another into the position shown in Fig. 6, in which the flange 7 lies in the space within the sleeve 13 which is delimited outwardly by the bead 18, and the flange 4 lies in the space within the sleeve 13 which is delimited outwardly by the bead 18, and the flange 4 lies in the space in the extension 20 which is delimited outwardly by the bead 22. When the containers 2 and 3 are moved toward one another, their flanges act as plungers in the sleeve 13 and the extension 20, respectively, although a not inconsiderable force is required to effect the movement past the bead 18 and 22, respectively, so that such displacement cannot be made unintentionally. The displacement past the beads 18 and 22 is possible because the connector 1 is made of some suitable resilient material, such as plastic. During the said displacement movement, the piercing member 15 will pierce the closure 8, and the combined connecting and piercing member 16 will pierce the closure 5 so that the containers 2 and 3 will communicate with one another via the passage 17. In this manner, liquid can be transferred from one container to the other. If the connecting member 16 has the shape shown in Fig. 4, the collar 24 will lie, after the displacement, on the inner side of the closure 5, the grooves 25 establishing an air communication into the liquid container 3 to facilitate emptying thereof.

All of the steps comprised by an operating sequence during use of the assembly according to Fig. 1 are schematically shown in Fig. 7. In the starting position A, the containers 2 and 3 are in the retracted position relative to the connector 1, as shown also in Figs. 1 and 5. In the next step 8, the containers 2 and 3 are simultaneously or consecutively displaced toward one another, whereby the closures 5 and 8 are pierced and communication is established between the containers 2 and 3, as also shown in Fig. 6. In a subsequent step C, the plunger 10 in the container 2 is retracted, whereby the liquid in the container 2 is transferred by suction to the container 2. In step D, the sleeve 13 then is moved into its retracted position relative to the container 2, whereby the piercing member 15 is retracted from its position piercing the closure 8. In this manner, the drug component and the liquid can be mixed in the container 2 without any risk that the operator is exposed to the mixture since the closure 8 closes itself completely at the point where it has previously been pierced by the piercing member 15. When the mixture within the container 2 is ready for administration, the extension 20 is removed in step E from the sleeve 13 by breaking away the portions 21. Before then, the container 3 has been pushed into its retracted position relative to the extension 20, or been completely removed therefrom.

After the extension 20 has been broken away, the connecting member 16 is exposed to be connected in step F to some suitable means for administration of the mixture in the container 2. This means may be, for example, a cannula, a catheter, a probe, or an infusion device having a connecting member complementary to the connecting member 16. After these connecting members have been connected together, the sleeve 13 is again moved into its inserted position relative to the container 2, as shown in step G of Fig. 7. Administration is then effected by inserting the plunger 10 into the container 2.

Figs. 8 and 9 illustrate an alternative embodiment of the connector 1 in which the extension 20 is missing and in which there is no sharp cutting edge for piercing on the connecting member 16. Fig. 8 shows the connector 1 in its retracted position relative to the container 2, and in Fig. 9 the connector is shown in its inserted position relative to the container 2, the piercing member 15 having pierced the closure 8 so that communication is established, via the passage 17, between the container 2 and the free end of the connecting member 16.

The handling of the connector according to Figs. 8 and 9 is illustrated in Fig. 10. In a first step A a conventional ampoule containing a predetermined amount of a solvent, is broken. In step B, an external means 26 is attached to the connecting member 16 of the connector. The external means 26 is a cannulla. A needle protector 27 which may be used for protecting the cannula against contamination and physical damage, is also removed. In step C, the connector 1 is moved into the inserted position relative to the container 2, and communication is established between the cannula 26 and the container 2 via the passage 17. In a subsequent step D, solvent is sucked from the ampoule into the drug container 2 by retracting the plunger 10 in the container 2. After that, the container 2 is closed in step E by moving the connector 1 into its retracted position in which the piercing member 15 does not pierce the closure 8 of the container 2. In position E, a drug in solid form can be left for dissolution. The cannula 26 may also be removed and replaced by some other means for administration. When the device is to be used, the connector 1 is again moved, in step F, from the retracted to the inserted position so that communication is established between the container 2 and the means for administration.

Although the device according to the invention has been described above in its application to dry, cytostatic drugs, a person skilled in the art will appreciate that also other toxic substances, e.g. other drugs which may be in liquid or dry form and which have other pharmacological effects, can be enclosed within the container 2.

Modifications of the embodiments described above are, of course, conceivable. Thus, the beads 19, 22 and 23 can be replaced by other positioning means, while the bead 18 preferably is a continuous bead. Moreover, the extension 20 may serve its function without being sleeve-shaped, and to facilitate its removal from the sleeve 13 it is also possible to reduce the thickness of the material.

Preferred embodiments of the invention have been described above and illustrated in the drawings, but it will be appreciated that these descriptions and drawings merely are intended to ilustrate the basic features of the invention and are not intended to restrict the scope of the invention and the appended claims.

## Claims

1. A disposable connector for a container (2) said container having at one end an opening which is closed by means of a pierceable closure (8) and which is surrounded by an outwardly directed flange (7), and having a plunger (10) movable within the container and closely engaging the container inner wall, said connector being formed as a single element comprising a sleeve (13) which is closed (14) at one end, which is open at its opposite end to receive the flanged container (2) end, and which has internal positioning means (18, 19) cooperating with the flange (7) and adapted to define an inserted and an extracted position of said sleeve relative to the flanged end of the container; a piercing member (15) provided at the closed end of the sleeve and directed towards the open sleeve end and adapted to pierce the closure (8) of the container upon displacement of the sleeve from the retracted to the inserted position

and a connecting member (16) provided at the closed sleeve end and directed opposite to said piercing member (15) and communicating therewith by means of a through passage (17), **characterised** in that said piercing member (15) and said connecting member (16) are integrally formed with the closed sleeve (13) out of the same material.

2. A connector as claimed in claim 1, **characterised** in that said positioning means (18, 19) comprise at least one inwardly directed bead (18).

3. A connector as claimed in claim 1 or 2, **characterised** in that said connecting member (16) also constitutes a piercing member for a pierceable closure (5) of a liquid container (3).

4. A connector as claimed in claim 3, **characterised** by a sleeve extension (20) extending in the same direction as said connecting member (16), for guiding the liquid container (3) toward said connecting member.

5. A connector as claimed in claim 4, **characterised** by internal positioning means (22, 23) in the sleeve extension (20) for defining an inserted and a retracted position of said extension relative to the liquid container (3).

6. A connector as claimed in claim 4 or 5, **characterised** in that said extension (20) is adapted to be broken away from the sleeve (13).

7. A connector as claimed in any one of claims 1-6, **characterised** by at least one axial groove (25) in the inner part of said connecting member (16).

8. A connector as claimed in claim 7, **characterised** in that said connecting member (16) has an outer part in the form of a truncated cone, into the narrow end of which the through passage (17) opens and forms a sharp edge, and an inner substantially cylindrical part in which the axial groove or grooves (25) are located.

9. A disposable assembly, **characterised** by a container (2) having at one end an opening which is closed by means of a pierceable closure (8) and which is surrounded by an outwardly directed flange (7), and having a plunger (10) movable within the container and closely engaging the container inner wall, a drug component being enclosed between said plunger and said closure, for mixing with a liquid prior to administration; a second container (3) for said liquid, said container having at one end an opening which is closed by a pierceable closure (5) and which is surrounded by an outwardly directed flange (4); and a connector, as claimed in claim 1, for connection of the drug container (2) first to the liquid container (3) for sucking up liquid in said drug container by retraction of the plunger therein, and then to a means for administration of the drug component/liquid mixture, said connector comprising an extension (20) of said sleeve (13), said extension extending in the same direction as said connecting member (16) and adapted to guide said liquid container toward said connecting member (16).

## Patentansprüche

1. Wegwerfbarer Verbinder für einen Behälter (2), welcher Behälter an einem Ende eine Öffnung besitzt, die mit Hilfe eines durchstoßbaren Verschlusses (8) verschlossen ist und die von einem nach außen gerichteten Flansch (7) umgeben ist, und einen innerhalb des Behälters verschiebbaren sowie an der Behälterinnenwand eng anliegenden Kolben (10) aufweist, wobei der Verbinder als Einzelelement ausgebildet ist, das aus einer Manschette (13) besteht, die an einem Ende geschlossen ist, am gegenüberliegenden Ende zur Aufnahme des mit dem Flansch versehenen Endes des Behälters (2) offen ist und die innenliegende Positionierelemente (18,19) aufweist, welche mit dem Flansch (7) zusammenwirken und zur Bestimmung einer aufgeschobenen und einer abgezogenen Lage der Manschette bezüglich des mit dem Flansch versehenen Behälterendes ausgebildet sind, wobei am geschlossenen Ende der Manschette ein Durchstoßglied (15), das zum offenen Manschettenende gerichtet und zum Durchstoßen des Verschlusses (8) des Behälters bei Verschiebung der Manschette aus der abgezogenen in die aufgeschobene Lage ausgebildet ist, und ein Anschlußglied (16) vorgesehen sind, das zum Durchstoßglied (15) entgegen gesetzt gerichtet ist und mit diesem mittels eines Durchgangskanales (17) in Verbindung steht, dadurch gekennzeichnet, daß das Durchstoßglied (15) und das Anschlußglied (16) einstückig mit der geschlossenen Manschette (13) aus demselben Material gebildet sind.

2. Verbinder nach Anspruch 1, dadurch gekennzeichnet, daß die Positionierelemente (18,19) aus wenigstens einem nach innen gerichteten Wulst (18) bestehen.

3. Verbinder nach Anspruch 1 oder 2, dadurch gekennzeichnet, daß das Anschlußglied (16) ebenfalls ein Durchstoßglied für einen durchstoßbaren Verschluß (5) eines Flüssigkeitsbehälters (3) darstellt.

4. Verbinder nach Anspruch 3, gekennzeichnet durch eine Manschettenverlängerung (20), die sich gleich weit wie das Anschlußglied (16) in dieselbe Richtung erstreckt und zur Führung des Flüssigkeitsbehälters (3) zum Anschlußglied dient.

5. Verbinder nach Anspruch 4, gekennzeichnet durch innenliegende Positionierelemente (22,23) in der Manschettenverlängerung (20) zur Bestimmung einer aufgeschobenen und einer abgezogenen Lage der Verlängerung bezüglich des Flüssigkeitsbehälters.

6. Verbinder nach Anspruch 4 oder 5, dadurch gekennzeichnet, daß die Verlängerung (20) zum Abbrechen von der Manschette (13) ausgebildet ist.

7. Verbinder nach den Ansprüchen 1 bis 6, gekennzeichnet durch wenigstens eine axiale Nut (25) im Innenteil des Anschlußgliedes (16).

8. Verbinder nach Anspruch 7, dadurch gekennzeichnet, daß das Anschlußglied (16) einen Außenteil in Form eines Kegelstumpfes, an dessen schmälerem Ende der Durchgangskanal (17) mündet und eine scharfe Kante bildet, und im wesentlichen zylindrischen Innenteil aufweist, in dem die axiale Nut bzw. axialen Nuten (25) angeordnet sind.

9.Wegwerfeinheit, gekennzeichnet durch einen Behälter (2), der an einem Ende eine Öffnung besitzt, die mit Hilfe eines durchstoßbaren Verschlusses (8) verschlossen ist und die von einem nach außen gerichteten Flansch (7) umgeben ist, und einen innerhalb des Behälters verschiebbaren sowie an der Behälterinnenwand eng anliegenden Kolben (10) aufweist, wobei zwischen dem Kolben und dem Verschluß eine Arzneimittelkomponente zum Mischen mit einer Flüssigkeit vor der Verabreichung eingeschlossen ist, durch einen zweiten Behälter (3) für die Flüssigkeit, der an einem Ende eine Öffnung besitzt, die mit einem durchstoßbaren Verschlusses (5) verschlossen ist und die von einem nach außen gerichteten Flansch (4) umgeben ist, durch einen Verbinder nach Anspruch 1, zum Verbinden des Arzneimittelbehälters (2) zuerst mit dem Flüssigkeitsbehälter (3) zum Ansaugen der Flüssigkeit in den Arzneimittelbehälter durch Zurückziehen des Kolbens in demselben und anschließend mit einer Einrichtung zur Verabreichung des Arzneimittelkomponenten/Flüssigkeits-Gemisches, wobei der Verbinder eine Verlängerung (20) der Manschette (13) aufweist, welche Verlängerung sich gleich weit wie das Anschlußglied (16) in dieselbe Richtung erstreckt und zur Führung des Flüssigkeitsbehälters zum Anschlußglied (16) ausgebildet ist.

**Revendications**

1. Raccord jetable pour un récipient (2), ledit récipient ayant à une première extrémité une ouverture qui est fermée à l'aide d'une fermeture perforable (8) et qui est entourée par un rebord (7) orienté vers l'extérieur, et ayant un piston (10) mobile à l'intérieur du récipient et venant étroitement au contact de la paroi interne du récipient, ledit raccord se présentant sous la forme d'une élément unique comprenant un manchon (13) qui est fermé (14) à urne première extrémité, qui est ouvert à son extrémité opposée pour recevoir le récipient (2) à rebord, et qui a des moyens intérieurs de positionnement (18, 19) coopérant avec le rebord (7) et conçus pour définir une position avancée et une position en retrait dudit manchon par rapport à l'extrémité à rebord du récipient; un organe de perforation (15) disposé à l'extrémité fermée du manchon et orienté vers l'extrémité ouverture du manchon et servant à perforer la fermeture (8) du récipient lorsque le manchon passe de la position en retrait à la position avancée, et un organe de raccordement (16) disposé à l'extrémité fermée du manchon et orienté en sens contraire dudit organe de perforation (15) et communiquant avec celui-ci par l'intermédiaire d'un passage traversant (17), caractérisé en ce que ledit organe de perforation (15) et ledit organe de raccordement (16) font corps avec le manchon fermé (13) réalisé dans la même matière.

2. Raccord selon la revendication 1, caractérisé en ce que lesdits moyens de positionnement (18, 19) comprennent au moins un épaulement (18) orienté vers l'intérieur.

3. Raccord selon la revendication 1 ou 2, caractérisé en ce que ledit organe de raccordement (16) constitue également un organe de perforation pour une fermeture perforable (5) d'un récipient (3) de liquide.

4. Raccord selon la revendication 3, caractérisé par un prolongement (20) de manchon s'étendant dans le même sens que ledit organe de raccordement (16), pour guider le récipient (3) de liquide vers ledit organe de raccordement.

5. Raccord selon la revendication 4, caractérisé par des moyens intérieurs de positionnement (22, 23) situés dans le prolongement (20) de manchon pour définir une position avancée et une position en retrait dudit prolongement par rapport au récipient (3) de liquide.

6. Raccord selon la revendication 4 ou 5, caractérisé en ce que ledit prolongement (20) est conçu pour se détacher du manchon (13) par cassure.

7. Raccord selon l'une quelconque des revendication 1 à 6, caractérisé par au moins une gorge axiale (25) dans la partie interne dudit organe de raccordement (16).

8. Raccord selon la revendication 7, caractérisé en ce que ledit organe de raccordement (16) a une partie extérieure de forme tronconique, dans l'extrémité étroite de laquelle le passage traversant (17) débouche et forme une arête vive, et une partie intérieure sensiblement cylindrique dans laquelle se trouve(nt) la/les gorge(s) (25).

9. Système jetable, caractérisé par un récipient (2) ayant à une première extrémité une ouverture qui est fermée à l'aide d'une fermeture perforable (8) et qui est entourée par un rebord (7) orienté vers l'extérieur, et ayant un piston (10) mobile à l'intérieur du récipient et venant étroitement au contact de la paroi interne du récipient, un constituant de médicament étant enfermé entre ledit piston et ladite fermeture pour être mélangé avec un liquide avant d'être administré; un second récipient (3) pour ledit liquide, ledit récipient ayant à une première extrémité une ouverture qui est fermée par une fermeture perforable (5) et qui est entourée par un rebord (4) orienté vers l'extérieur; et un raccord selon la revendication 1, pour raccorder le récipient (2) de médicament d'abord au récipient (3) de liquide afin d'aspirer le liquide dans ledit récipient de médicament en faisant reculer le piston situé dans celui-ci, puis à moyen pour l'administration dudit mélange de constituant de médicament et de liquide, ledit raccord comportant un prolongement (20) dudit manchon (13), ledit prolongement s'étendant dans le même sens que ledit organe de raccordement (16) et servant à guider ledit récipient de liquide vers ledit organe de raccordement (16).

Fig.1

Fig.2

Fig.3

Fig.4

Fig.5

Fig.6

Fig.8

Fig.9

## Fig.7

Fig. 10